# EUROPÄISCHE PATENTSCHRIFT

(11) **EP 0 687 666 B1**
(45) Veröffentlichungstag und Bekanntmachung des Hinweises auf die Patenterteilung: **03.12.1997**
(21) Anmeldenummer: 95108485.4
(22) Anmeldetag: 02.06.1995
(51) Int. Cl.: C07C 68/08, C07C 69/96

(54) **Verfahren zur Reinigung von Diphenylcarbonat**
Process for purifying diphenyle carbonate
Procédé de purification du carbonate de déphényle

(30) Priorität: 15.06.1994 DE 4420778
(43) Veröffentlichungstag der Anmeldung: 20.12.1995
(73) Patentinhaber: BAYER AG, 51368 Leverkusen (DE)
(72) Erfinder: Buysch, Hans-Josef, Dr., D-47809 Krefeld (DE); Mendoza-Frohn, Christine, Dr., D-40699 Erkrath (DE); Rechner, Johann, Dr., D-47800 Krefeld (DE); Schön, Norbert, Dr., D-64289 Darmstadt (DE)

(56) Entgegenhaltungen:
- EP-A- 0 636 620

## Beschreibung

Die Erfindung hat ein Verfahren zur Reinigung von Diphenylcarbonat (DPC) aus Rohprodukten der Diphenylcarbonatherstellung zum Gegenstand, bei dem man Rohprodukte einsetzt, deren destillierbarer Anteil zu mehr als 70 Gew.-% aus Diphenylcarbonat besteht und sie fraktioniert aus der Schmelze kristallisiert (fraktionierende Schmelzkristallisation).

Diphenylcarbonat kann z.B. durch Umsetzung von Phenol mit Phosgen in Gegenwart von äquimolaren Mengen wäßriger Natronlauge, durch Phosgenieren von Phenol in Gegenwart von Katalysatoren, Umesterung von Phenol mit Dimethylcarbonat oder oxidative Carbonylierung von Phenol hergestellt werden.

Die Herstellung und Reinigung von Diphenylcarbonat kann durch Destillation, Extraktion von Phenol mit Wasser oder durch Kristallisation erfolgen (BR-A 1 096 936, US-A 4 013 702, US-A 5 239 106).

Ein Verfahren zur Kristallisation wird z.B. in der US-A 5 239 106 beschrieben. Es weist verschiedene Nachteile auf:

Da bei der Kristallisation kein reines Diphenylcarbonat, sondern ein Addukt mit Phenol erhalten wird, muß das Kristallisat in einem weiteren Verfahrensschritt destillativ gereinigt werden. Nach diesem Verfahren können nur solche Rohgemische aufgearbeitet werden, die einen geringen Feststoffanteil, d.h. geringen Diphenylcarbonatgehalt, besitzen, weil die Gemische sonst nicht mehr zu handhaben sind.

Daher darf das Eduktmaterial für die Kristallisation nur Gehalte an Diphenylcarbonat von weniger als 70 Gew.-%, besser nicht mehr als 50 Gew.-% (l.c. Sp. 2, Zeilen 40 bis 53 und Beispiel 5) aufweisen, damit der Kristallbrei noch ausreichend durch Filtration von der Mutterlauge befreit werden kann.

Dies ist umso weniger vorteilhaft, als heute bei technisch ausgeführten Synthesen Rohprodukte erhalten werden, die deutlich höhere Gehalte an Diphenylcarbonat aufweisen.

Es wurde nun ein Verfahren zur Reinigung von Diphenylcarbonat (DPC) aus Rohprodukten der Diphenylcarbonatherstellung mit hohen Diphenylcarbonatgehalten von über 70 Gew.-%, bezogen auf den destillierbaren Anteil, durch fraktionierte Schmelzkristallisation gefunden. Überraschenderweise eignet sich das erfindungsgemäße Verfahren zur Reinigung von Diphenylcarbonat-Rohprodukten aus unterschiedlichen Herstellungsverfahren, obwohl diese völlig andersartige Verunreinigungen und Nebenprodukte enthalten.

Gegenstand der Erfindung ist ein Verfahren zur Reinigung von Diphenylcarbonat durch Kristallisation aus Rohprodukten der Diphenylcarbonatherstellung mit hohen Gehalten an Diphenylcarbonat von über 70 Gew.-%, bezogen auf den destillierbaren Anteil, durch fraktionierende Schmelzkristallisation, dadurch gekennzeichnet, daß die zu reinigende Schmelze im Bereich von 85 bis 45°C, bevorzugt 80 bis 48°C, mit einer Kühlrate von 20 bis 0,1°C/h, bevorzugt 10 bis 0,5°C/h, abgekühlt wird, bei der niedrigsten Kühlmitteltemperatur vor der Abtrennung der Restschmelze eine Haltezeit von 0 bis 100 Minuten, bevorzugt 1 bis 70 Minuten, eingehalten wird, anschließend durch Aufheizen mit einer Heizrate von 20 bis 0,1°C/h, bevorzugt 10 bis 0,5°C/h, bis zu einer Endtemperatur von 70 bis 80°C, bevorzugt 72 bis 79,5°C, das Kristallisat abgeschmolzen wird und während des Aufheizens gegebenenfalls weitere Schmelzanteile mit Verunreinigungen an Haltepunkten oder ohne Unterbrechung des Aufheizens, von der bei höherer Temperatur anfallenden reinen DPC-Schmelze abgetrennt werden.

Das erfindungsgemäße Verfahren läßt sich zur Reinigung von Diphenylcarbonat-Rohprodukten der im folgenden aufgeführten Verfahren einsetzen.

Bei der Phasengrenzflächenphosgenierung von Phenol entsteht ein Rohprodukt mit über 99 Gew.-% DPC, das als Nebenprodukte Chlorameisensäurephenylester enthält.

Aus der katalytischen Direktphosgenierung von Phenol erhält man ein Diphenylcarbonat-Rohprodukt mit >95 Gew.-% DPC. Als Nebenprodukte entstehen auch hier Chlorameisensäurephenylester und Salicylsäurephenylester.

Die Umesterung von Dimethylcarbonat (DMC) mit Phenol liefert beispielsweise Reaktionsprodukte mit 0 bis 2 Gew.-% DMC, 1,5 bis 10 Gew.-% Phenol, 1 bis 20 Gew.-% Methylphenylcarbonat (MPC), 70 bis 98 Gew.-% Diphenylcarbonat und in ppm-Konzentrationen Nebenprodukte wie Salicylsäurephenyl-, Salicylsäuremethylester und Anisol. Zusätzlich enthält dieses Gemisch noch gegebenenfalls Katalysatoren oder deren Reste, d.h. Titan- oder Zinnverbindungen.

Die oxidative Carbonylierung von Phenol ergibt je nach Reaktionsbedingungen, Laufzeiten und Katalysatorkonzentrationen DPC-Rohlösungen mit 20 bis 90 Gew.-% Phenol, 10 bis 80 Gew.-% DPC und als Nebenprodukte in geringen Konzentrationen noch Nebenprodukte wie Salicylsäurephenylester, o-Phenylphenol und Diphenylether. Zusätzlich enthalten diese Gemische noch die Komponenten des Katalysatorsystems, d.h. beispielsweise Tetrabutylammoniumbromid, Natriumphenolat, Mangan- oder Co- oder Cu- und Palladiumverbindungen.

Auch aus den verdünnten Lösungen dieses Verfahrens kann man nach Abdestillieren des Phenols oder einfachen Vorkristallisationen Rohlösungen mit 70 bis 85 Gew.-% DPC erhalten, mit denen sich das erfindungsgemäße Reinigungsverfahren vorteilhaft durchführen läßt.

Die Menge an Diphenylcarbonat im destillierbaren Anteil des Rohproduktes beträgt also über 70 Gew.-%, vorzugsweise über 75 Gew.-%, besonders bevorzugt über 80 Gew.-%.

Die bei der erfindungsgemäßen Kristallisation erhaltenen Produkte können problemlos gehandhabt werden; sie sind keine Phenol/Diphenylcarbonat-Addukte und müssen dementsprechend nicht durch zusätzliche Destillation zum reinen Diphenylcarbonat geführt werden.

Das erfindungsgemäße Verfahren der fraktionierenden Schmelzkristallisation von verunreinigtem Diphenylcarbonat kann sowohl diskontinuierlich als auch kontinuierlich ein- oder mehrstufig durchgeführt werden.

Für die erfindungsgemäße Reinigung von Diphenylcarbonat können Kristallisationsverfahren eingesetzt werden, wie sie beispielsweise in Chem. Ing. Techn. 57, (1985), 91, Chem. Ing. Techn. 63, (1991), 881, Ullmann's Encyclopedia 4. Aufl. Bd. 2, S. 672 ff und 5. Aufl. Bd. 32, S. 3 ff oder im Prospekt zur fraktionierten Kristallisation der Firma Sulzer vom August 1992 beschrieben sind.

Zur Reinigung von Diphenylcarabonat sind statische und dynamische Suspensions-und Schichtkristallisationsverfahren einsetzbar, bevorzugt dynamische Verfahren.

Zur Durchführung des erfindungsgemäßen Verfahrens können z.B. Rohrbündel-Kristallisatoren oder modifizierte Plattenwärmeaustauscher unterschiedlicher Konstruktion mit oder ohne Umpumpen der Schmelze, mit und ohne Anwendung von Pulsationen bzw. mit und ohne Unterteilung der Rohre in Segmente mit separater Ausspeisung verwendet werden. Weiterhin können Fallfilmkristallisatoren unterschiedlicher Bauweise, z.B. die aus der EP-A 218 545 bekannten, eingesetzt werden. Weitere einsetzbare Apparate sind Blasensäulenkristallisatoren, Kristallisierwalzen und -bänder. Weitere Einzelheiten einer kontinuierlich arbeitenden, geeigneten Kristallisationseinrichtung finden sich in der EP-A 521 499.

Bevorzugt werden für das erfindungsgemäße Verfahren Rohrbündel-Kristallisatoren, Plattenwärmeaustauscher unterschiedlicher Konstruktion mit und ohne Umpumpen der Schmelze oder Fallfilmkristallisatoren eingesetzt.

Der Kristallisationsvorgang im erfindungsgemäßen Verfahren zur Reinigung eines Diphenylcarbonates aus der Herstellung, entsprechend den o.a. Verfahren kann sowohl durch spontane Keimbildung als auch durch eine definierte Zufuhr von Kristallisationskeimen (Impfung) eingeleitet werden. Vorzugsweise wird die Kristallbildung durch Kristallkeime eingeleitet.

Das erfindungsgemäße Verfahren zur Gewinnung von gereinigtem Diphenylcarbonat durch Schmelzkristallisation kann mit destillativen Reinigungsverfahren verbunden werden. So ist es möglich, vor Anwendung der Schmelzkristallisation, insbesondere bei der Abtrennung von Diphenylcarbonat aus gegebenenfalls katalysatorhaltigen Reaktionslösungen, eine Destillation von Diphenylcarbonat durchzuführen, dann erfindungsgemäß eine Schmelzkristallisation anzuschließen.

Ferner kann man nach erfolgter Kristallisation auch geringe Mengen an Leichtsieder durch einfache Destillation aus dem gereinigten Diphenylcarbonat entfernen.

Auch kann man vorhandene Katalysatoren durch Ausfällen und Filtration zu einem großen Teil vom Diphenylcarbonat abtrennen.

Man kann das erfindungsgemäße Verfahren auch mit anderen einfachen Kristallisationsverfahren kombinieren, beispielsweise dem in der US-A 5 239 106 beschriebenen, wobei man schon an Diphenylcarbonat angereicherte Gemische erhält, die dann nach dem erfindungsgemäßen Verfahren weiter gereinigt werden.

Im Falle der Ausführung des erfindungsgemäßen Verfahrens in Rohrbündel-Kristallisatoren oder Plattenwärmeaustauschern wird die zu reinigende Schmelze im Bereich von 85 bis 45°C, bevorzugt 80 bis 48°C mit einer Kühlrate von 20 bis 0,1°C/h, bevorzugt 10 bis 0,5°C/h abgekühlt. Während dieser Abkühlphase wird der Kristallisationsvorgang durch spontane Keimbildung oder durch definierte Keimzufuhr (Impfung), bevorzugt durch Impfung, eingeleitet. Bei der niedrigsten Temperatur des Kühlmediums wird gegebenenfalls vor der Abtrennung der Restschmelze eine Haltezeit von bis zu 100 Minuten eingehalten. Die Haltezeit beträgt daher unter Berücksichtigung beider Varianten 0 bis 100 Minuten, bevorzugt 1 bis 70 Minuten. Danach erfolgt die Abtrennung der Restschmelze, und das Kristallisat wird durch Aufheizen mit einer Heizrate von 20 bis 0,1°C/h, bevorzugt 10 bis 0,5°C/h, bis zu einer Endtemperatur von 70 bis 80°C, bevorzugt 72 bis 79,5°C weiter gereinigt.

Zur Verbesserung der Reinigungsleistung können während dieser Aufheizphase gegebenenfalls weitere Haltepunkte zur Abtrennung weiterer Schmelzeanteile dienen und bis zu diesen Haltepunkten abgeschmolzene Verunreinigungen zusammen mit der Schmelze entfernt werden. In einer weiteren Ausführungsform kann während des Aufheizvorgangs, ohne Unterbrechung der Aufheizung, die zunächst anfallende Schmelze mit den ausgeschwitzten restlichen Verunreinigungen abgetrennt und somit von der bei höherer Temperatur anfallenden reinen DPC-Schmelze getrennt werden.

Das erfindungsgemäß gereinigte Diphenylcarbonat kann z.B. zur Herstellung von Umesterungsprodukten wie Polycarbonaten (aus z.B. Bisphenol-A) verwendet werden.

### Beispiele

In den folgenden Beispielen werden fraktionierende Schmelzkristallisationen durchgeführt. Die hergestellte Schmelze des Diphenylcarbonates wird in ein senkrecht stehendes ummanteltes Rohr von 150 cm Höhe und ca. 3 cm Innendurchmesser gegeben und ausgehend von 80 bis 85°C entsprechend einer bestimmten Abkühlrate (°C/h) abgekühlt. Die Schmelze wird dann mit Diphenylcarbonatkristallen geimpft. Nachdem sich eine Kristallschicht gebildet hat, läßt man die Restschmelze ablaufen und beginnt dann durch Aufheizen mit einer bestimmten Rate mit dem "Schwitzen" der Kristalle, wobei weitere Schmelze abtropft. Diesen Vorgang beendet man bei einer Temperatur von 45 bis 70°C, heizt dann weiter auf, schmilzt die im Rohr verbliebenen Kristalle auf und fängt diese Schmelze von gereinigtem Diphenylcarbonat in einem getrennten Gefäß auf.

### Beispiel 1

Ein Reaktionsprodukt aus der Umesterung von Phenol mit Dimethylcarbonat enthaltend etwa
1 Gew.-% Dimethylcarbonat
5 Gew.-% Methylphenylcarbonat
5 Gew.-% Phenol
84 Gew.-% Diphenylcarbonat
5 Gew.-% Titantetraphenolat
wurde durch Abdestillieren der leichter flüchtigen Komponenten auf eine Zusammensetzung von
94 Gew.-% Diphenylcarbonat
6 Gew.-% Titantetraphenolat
gebracht, in den Rohrkristaller gefüllt, zunächst auf 78,0°C temperiert und dann mit 2,0°C/h abgekühlt. Bei 76,8°C wurde die Schmelze mit einigen Kristallen Diphenylcarbonat angeimpft. Als die Schmelze 70,2°C erreichte, wurde sie abgelassen und dann das Heizmedium mit 2°C/h wieder aufgeheizt. Nach Erreichen von 78,1°C wurde die im Rohr verbliebene Kristallmasse abgeschmolzen und separat aufgefangen. Sie enthielt nach dieser ersten Kristallisierstufe nur noch 0,3 Gew.-% Katalysator bei einer Kristallisationsausbeute von 55 % und nach einer 2 analogen Stufe nur noch 330 ppm Katalysator.

### Beispiel 2

Das Reaktionsprodukt mit der im Beispiel 1 genannten Zusammensetzung wurde vom Katalysator abdestilliert und mit einer Zusammensetzung von ca.
89 Gew.-% Diphenylcarbonat
5 Gew.-% Methylphenylcarbonat
5 Gew.-% Phenol
1 Gew.-% Dimethylcarbonat
140 ppm Phenylsalicylat
in die Kristallisation eingesetzt. Beginnend bei 70°C wurde mit 5°C/h abgekühlt. Als die Schmelze 51,0°C erreichte, wurde sie abgelassen und von da an wieder aufgeheizt mit 3°C/h. Bei Erreichen von 75,7°C in der ablaufenden Schmelze wurde der im Rohr verbliebene Kristallbelag abgeschmolzen und separat aufgefangen. Er betrug 55,2 % des eingesetzten Produktes und enthielt
98,65 Gew.-% Diphenylcarbonat
0,61 Gew.-% Methylphenylcarbonat
0,72 Gew.-% Phenol
0,02 Gew.-% Dimethylcarbonat
- Phenylsalicylat (< 10 ppm)
Die geringen Mengen an Leichtsiedern lassen sich durch Andestillieren leicht auf kritische Werte < 0,02 % bringen.

### Beispiel 3

Das Reaktionsprodukt mit der im Beispiel 1 genannten Zusammensetzung wurde auf 60 bis 70°C abgekühlt und der ausgefallene Katalysator Titantetraphenolat abgesaugt.

Man erhielt ein Gemisch mit ca.
89 Gew.-% Diphenylcarbonat
5 Gew.-% Methylphenylcarbonat
5 Gew.-% Phenol
1 Gew.-% Dimethylcarbonat
0,01 Gew.-% Titantetraphenolat
121 ppm Phenylsalicylat
das in die Kristallisation eingesetzt und von 72,0°C mit einer Rate von 2°C/h auf 62,0°C abgekühlt wurde. Nachdem die Mutterlauge abgelaufen war, wurde mit 2°C/h wieder aufgeheizt, bis die ablaufende Schmelze 78,0°C heiß war. Dann wurde das im Rohr verbliebene Kristallisat abgeschmolzen und separat aufgefangen.

Es betrug 42 % der eingesetzten Menge und enthielt
99,50 Gew.-% Diphenylcarbonat
0,18 Gew.-% Methylphenylcarbonat
0,31 Gew.-% Phenol und
10 ppm Titantetraphenolat
Das so gereinigte Produkt wird nach dem gleichen Verfahren noch einmal kristallisiert. Man erhält ein Diphenylcarbonat mit einer Reinheit von 99,98 Gew.-%, das unter < 3 ppm Titantetraphenolat enthält.

### Beispiel 4

Beispiel 3 wurde wiederholt mit dem Unterschied, daß die Abkühlrate von 70°C auf 50,9°C 5°C/h und die Aufheizrate von 50,9°C auf 76,1°C 3°C/h betrug, und daß bei 68,5°C mit Diphenylcarbonat angeimpft wurde. Bei Erreichen einer Schmelztemperatur von 76,1°C wurden die im Rohr verbliebenen Kristalle aufgeschmolzen und die Schmelze separat aufgefangen. Sie betrug 48,3 Gew.-% des eingesetzten Materials und enthielt
98,80 Gew.-% Diphenylcarbonat
0,42 Gew.-% Methylphenylcarbonat
0,81 Gew.-% Phenol
0,02 Gew.-% Dimethylcarbonat
13 ppm Titantetraphenolat
Phenylsalicylat nicht nachweisbar (< 10 ppm)

### Vergleichsbeispiel 1

Das Beispiel 4 der US-A 5 239 106 wurde nachgearbeitet:
Das Gemisch aus 54,1 Gew.-% Diphenylcarbonat, 44,6 Gew.-% Phenol und 1,3 Gew.-% Phenylsalicylat wurde aufgeschmolzen, homogenisiert und unter Rühren in 60 Minuten von 100°C auf 44°C abgekühlt, wobei ein dicker Kristallbrei entstand. Dieser wurde auf einer mit Thermostat exakt auf 44°C temperierten Fritte abgesaugt und sehr gut abgepreßt. Nachdem keine Mutterlauge mehr abtropfte, wurden die Kristalle ausgewogen. Man erhielt 43,2 g Kristalle. Diese wurden zur Homogenisierung wieder aufgeschmolzen und eine Probe der Schmelze analysiert.
- Sie bestand aus: 30,63 Gew.-% Phenol
69,11 Gew.-% Diphenylcarbonat
0,26 Gew.-% Phenylsalicylat.

Das Verfahren der US-A 5 239 106 liefert also deutlich ungünstigere Ergebnisse.

### Vergleichsbeispiel 2

Ein Produkt der Zusammensetzung, wie es in Beispiel 4 der US-A 230 106 beschrieben wird, wurde analog Beispiel 4 im Rohrkristaller mit 2°C/h von 70°C auf 44°C abgekühlt und 1 Stunde bei dieser Temperatur belassen. Dann ließ man die Schmelze vollständig abtropfen und führte schließlich die Kristalle im Rohr in eine Schmelze über. Sie betrug 36 % der gesamten eingesetzten Menge und enthielt
65,1 % Diphenylcarbonat (44 % der eingesetzten Diphenylcarbonatmenge)
34,8 % Phenol
400 ppm Phenylsalicylat

### Aus den Beispielen ergibt sich:

- Nach dem erfindungsgemäßen Verfahren können auch hochkonzentrierte, über 70 Gew.-% an Diphenylcarbonat enthaltende Gemische problemlos gehandhabt und durch Kristallisation gereinigt werden (Beispiel 1 bis 4).
- Selbst nach einstufiger Kristallisation werden schon sehr reine Produkte erhalten (Beispiel 3)
- Anschließende Phenoldestillation ist nicht erforderlich.
- Nebenprodukte (Phenylsalicylat) werden bis unter die Nachweisgrenze entfernt (Beispiel 2 bis 4; Vergl. Beispiel 1 und 2).
- Katalysatoren werden schon nach einstufiger Kristallisation auf sehr niedrige Werte, nach zweistufiger Kristallisation unter die Nachweisgrenze gebracht (Beispiel 3).
- Die Ausbeute an reinem Kristallisat ist deutlich höher als nach dem Stand der Technik (Vergleichsbeispiel 2 mit Vergleichsbeispielen 1 und 2).

### Beispiel 5

Eine Reaktionslösung aus der Direktcarbonylierung von Phenol mit einer Zusammensetzung von etwa
- 24 Gew.-%: Diphenylcarbonat
- 74 Gew.-%: Phenol
- 1,5 Gew.-%: Tetrabutylammoniumbromid
- 0,4 Gew.-%: Natriumphenolat
- 0,1 Gew.-%: Manganverbindung
- 200 ppm: Palladiumverbindung
sowie ca. 150 ppm Nebenprodukte wie Salicylsäurephenylester und o-Phenylphenol, wurde durch Abfiltrieren von Feststoffen und Abdestillieren von Phenol auf eine Zusammensetzung von
- 73,2 Gew.-%: Diphenylcarbonat
- 18,9 Gew.-%: Phenol
- 7,5 Gew.-%: Tetrabutylammoniumbromid
- 0,4 Gew.-%: Natriumphenolat
- 76 ppm: Manganverbindung
- 5 ppm: Palladiumverbindung
sowie ca. 500 ppm Nebenprodukte wie Salicylsäurephenylester und o-Phenylphenol, gebracht, in den Rohrkristaller gefüllt, zunächst auf 70°C temperiert und mit 2°C/h abgekühlt. Als die Schmelze 61°C erreicht hatte, wurde sie 30 Minuten bei dieser Temperatur gehalten, anschließend abgelassen und im Anschluß daran das Heizmedium mit 2°C/h wieder aufgeheizt. Nach Erreichen von 79°C schmolz die im Kristaller verbliebene Kristallmasse ab und wurde separat aufgefangen. Sie enthielt nach dieser ersten Kristallisierstufe folgende Zusammensetzung:
- 97,5 Gew.-%: Diphenylcarbonat
- 2,0 Gew.-%: Phenol
- 0,5 Gew.-%: Tetrabutylammoniumbromid
- 6 ppm: Natriumphenolat
Pd-, Mn-Verbindungen waren nicht mehr nachweisbar. Nebenprodukte wie Salicylsäurephenylester und o-Phenylphenol lagen in Konzentrationen <10 ppm vor. Nach einer weiteren gleichen Kristallisationsstufe lag ein DPC-Gehalt von >99,8 Gew.-% vor.

### Beispiel 6

Eine Reaktionslösung aus der Direktcarbonylierung von Phenol mit etwa
- 75 Gew.-%: Diphenylcarbonat
- 23 Gew.-%: Phenol
- 1,5 Gew.-%: Tetrabutylammoniumbromid
- 0,4 Gew.-%: Natriumphenolat
- 0,1 Gew.-%: Manganverbindung
- 200 ppm: Palladiumverbindung
sowie ca. 210 ppm Nebenprodukte wie Salicylsäurephenylester und o-Phenylphenol, wurde durch Abfiltrieren von Feststoffen auf eine Zusammensetzung von
- 75,8 Gew.-%: Diphenylcarbonat
- 23,3 Gew.-%: Phenol
- 0,7 Gew.-%: Tetrabutylammoniumbromid
- 0,2 Gew.-%: Natriumphenolat
- 80 ppm: Manganverbindung
- 6 ppm: Palladiumverbindung
gebracht, in den Rohrkristaller gefüllt, zunächst auf 70°C temperiert und mit 2°C/h abgekühlt. Als die Schmelze 61 °C erreicht hatte, wurde sie abgelassen und das Heizmedium anschließend mit 2°C/h wieder aufgeheizt. Nach Erreichen von 79°C schmolz die im Kristaller verbliebene Kristallmasse ab und wurde separat aufgefangen. Sie enthielt nach dieser ersten Kristallisierstufe folgende Zusammensetzung:
- 98,6 Gew.-%: Diphenylcarbonat
- 1,1 Gew.-%: Phenol
- 0,3 Gew.-%: Tetrabutylammoniumbromid
- 4 ppm: Natriumphenolat
Pd-, Mn-Verbindungen waren nicht mehr nachweisbar. Nebenprodukte wie Salicylsäurephenylester und o-Phenylphenol lagen in Konzentrationen <8 ppm vor. Das so erhaltene Kristallisat wurde destilliert und besaß dann einen DPC-Gehalt von >99,9 Gew.-%.

### Beispiel 7

Ein Reaktionsprodukt aus der katalytischen Phosgenierung von Phenol enthaltend
- 75,6 Gew.-%: Diphenylcarbonat
- 23,5 Gew.-%: Phenol
- 0,9 Gew.-%: Chlorameisensäurephenylester
- 1090 ppm: Salicylsäurephenylester
wurde einer Kristallisation analog Beispiel 2 unterworfen. Dabei erhielt man ein gereinigtes Diphenylcarbonat folgender Zusammensetzung:
- 99,5 Gew.-%: Diphenylcarbonat
- 0,5 Gew.-%: Phenol
- <0,01 Gew.-%: Chlorameisensäurephenylester
- 50 ppm: Salicylsäurephenylester

Die restlichen Mengen Leichtsieder können durch einfaches Andestllieren unter die Nachweisgrenze entfernt werden.

## Patentansprüche

1. Verfahren zur Reinigung von Diphenylcarbonat durch Kristallisation, aus Rohprodukten der Diphenylcarbonatherstellung mit hohen Gehalten an Diphenylcarbonat von über 70 Gew.-%, bezogen auf den destillierbaren Anteil, durch fraktionierende Schmelzkristallisation, dadurch gekennzeichnet, daß die zu reinigende Schmelze im Bereich von 85 bis 45°C, bevorzugt 80 bis 48°C, mit einer Kühlrate von 20 bis 0,1°C/h, bevorzugt 10 bis 0,5°C/h, abgekühlt wird, bei der niedrigsten Kühlmitteltemperatur vor der Abtrennung der Restschmelze eine Haltezeit von 0 bis 100 Minuten, bevorzugt 1 bis 70 Minuten, eingehalten wird, anschließend durch Aufheizen mit einer Heizrate von 20 bis 0,1°C/h, bevorzugt 10 bis 0,5°C/h, bis zu einer Endtemperatur von 70 bis 80°C, bevorzugt 72 bis 79,5°C, das Kristallisat abgeschmolzen wird und während des Aufheizens gegebenenfalls weitere Schmelzanteile mit Verunreinigungen an Haltepunkten oder ohne Unterbrechung des Aufheizens, von der bei höherer Temperatur anfallenden reinen DPC-Schmelze abgetrennt werden.

## Claims

1. Process for the purification of diphenyl carbonate by crystallisation from crude products of diphenyl carbonate production which have elevated diphenyl carbonate contents of above 70 wt.%, relative to the distillable fraction, by fractionating melt crystallisation, characterised in that the melt to be purified is cooled in the range from 85 to 45°C, preferably from 80 to 48°C, at a cooling rate of 20 to 0.1°C/h, preferably of 10 to 0.5°C/h, a holding time of 0 to 100 minutes, preferably of 1 to 70 minutes, is maintained at the lowest coolant temperature before separation of the residual melt, the crystallized product is then melted by heating at a heating rate of 20 to 0.1°C/h, preferably of 10 to 0.5°C/h, to a final temperature of 70 to 80°C, preferably of 72 to 79.5°C and, during heating, further fractions of the melt with impurities are optionally separated at pauses or without interruption of heating from the pure DPC melt arising at a higher temperature.

## Revendications

1. Procédé de purification de carbonate de diphényle par cristallisation, à partir de produits bruts de la préparation du carbonate de diphényle avec des teneurs élevées en carbonate de diphényle supérieures à 70 % en poids, par rapport à la fraction distillable, par cristallisation fractionnée de masse fondue, caractérisé en ce qu'on refroidit la masse fondue à purifier dans l'intervalle de 85 à 45°C, de préférence entre 80 et 48°C, à une vitesse de refroidissement de 20 à 0,1°C/h, de préférence 10 à 0,5°C/h, où on maintient la température du milieu de refroidissement la plus basse avant la séparation de la masse fondue restante pendant 0 à 100 min, de préférence 1 à 70 min, ensuite par réchauffage avec une vitesse de chauffage de 20 à 0,1°C/h, de préférence 10 à 0,5°C/h, on refond jusqu'à une température finale de 70 à 80°C, de préférence 72 à 79,5°C le produit cristallisé et pendant le réchauffage, on sépare le cas échéant d'autres fractions fondues avec des impuretés aux points de maintien ou sans interrompre le chauffage, on sépare la masse fondue de DPC pur obtenue à une température supérieure.
